# EUROPEAN PATENT APPLICATION

(11) **EP 2 380 572 A1**
(43) Date of publication of application: **26.10.2011**
(21) Application number: 10194774.5
(22) Date of filing: 29.11.2006
(51) Int. Cl.: A61K 31/506, A61P 35/00

(54) **Cancer treatment method**

(30) Priority: 29.11.2005 US 740308 P
(62) Divisional of application: 06838636.6
(71) Applicant: Glaxosmithkline LLC, Philadelphia, PA 19102 (US)
(72) Inventor: Pandite, Arundathy Nirmalini, Durham, NC 27709-3398 (US); Whitehead, Bonnie F., Durham, NC 27709-3398 (US); Ho, Peter T.C., Collegeville, PA 19426 (US); Suttle, Albert Benjamin, Durham, NC 27709-3398 (US)
(74) Representative: Learoyd, Stephanie Anne

(57) **Abstract**

The present invention relates to a method of treating cancer in a mammal by administration of pyrimidine derivatives. In particular, the method relates to a method of treating cancer by administration of 5-[[4-[(2,3-Dimethyl-2H-indazol-6-yl)methylamino]-2-pyrimidinyl]amino]-2-methylbenzenesulfonamide or a salt or solvate thereof. In particular the cancer is thyroid cancer.

## Description

### FIELD OF THE INVENTION

The present invention relates to a method of treating cancer in a mammal by administration of pyrimidine derivatives. In particular, the method relates to a method of treating cancer by administration of 5-[[4-[(2,3-Dimethyl-2H-indazol-6-yl)methylamino]-2-pyrimidinyl]amino]-2-methylbenzenesulfonamide or a salt or solvate thereof.

### BACKGROUND OF THE INVENTION

Effective chemotherapy for cancer treatment is a continuing goal in the oncology field. Generally, cancer results from the deregulation of the normal processes that control cell division, differentiation and apoptotic cell death. Apoptosis (programmed cell death) plays essential roles in embryonic development and pathogenesis of various diseases, such as degenerative neuronal diseases, cardiovascular diseases and cancer.

In cancer, the growth of tumors has been shown to be dependent on angiogenesis. The progression of leukemias as well as the accumulation of fluid associated with malignant ascites and pleural effusions also involve pro-angiogenic factors. (See Folkmann, J., J. Nat'l. Cancer Inst., 82:4-6 (1990)). Consequently, the targeting of pro-angiogenic pathways is a strategy being widely pursued in order to provide new therapeutics in these areas of great, unmet medical need.

Central to the process of angiogenesis are vascular endothelial growth factor (VEGF) and its receptors, termed vascular endothelial growth factor receptor(s) (VEGFRs). Three protein tyrosine kinase receptors for VEGF have been identified: VEGFR1 (Flt-1), VEGFR2 (Flk-1 and KDR), and VEGFR3 (Flt-4). These receptors are involved in angiogenesis and participate in signal transduction. (Mustonen, T. et al J. Cell Biol. 129:895-898 (1995); Ferrara and Davis-Smyth, Endocrine Reviews, 18(1):4-25 (1997); McMahon, G., The Oncologist, 5(90001 ):3-10 (2000)).

Consequently, antagonism of the VEGFR kinase domain is believed to block phosphorylation of tyrosine residues and serve to disrupt initiation of angiogenesis, and other signaling pathways mediated by VEGF, thereby providing a potent treatment for many types of cancer.

### SUMMARY OF THE INVENTION

The present inventors have now identified novel cancer treatment methods which include administration of 5-[[4-[(2,3-Dimethyl-2H-indazol-6-yl)methylamino]-2-pyrimidinyl]amino]-2-methylbenzenesulfonamide or a salt or solvate thereof.

In one aspect of the present invention, there is provided a method of treating susceptible cancers in a mammal, comprising: administering to the mammal a compound of formula (1) 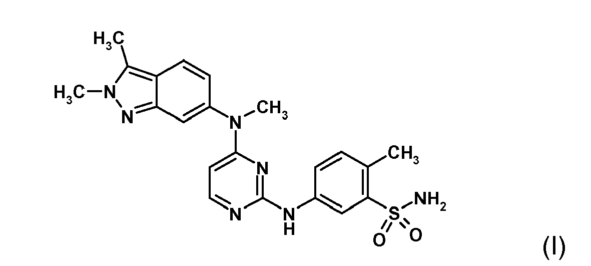 or a salt or solvate thereof.

### DETAILED DESCRIPTION OF THE INVENTION

As used herein the term "neoplasm" refers to an abnormal growth of cells or tissue and is understood to include benign, i.e., non-cancerous growths, and malignant, i.e., cancerous growths. The term "neoplastic" means of or related to a neoplasm.

As used herein, the term "effective amount" means that amount of a drug or pharmaceutical agent that will elicit the biological or medical response of a tissue, system, animal or human that is being sought, for instance, by a researcher or clinician. Furthermore, the term "therapeutically effective amount" means any amount which, as compared to a corresponding subject who has not received such amount, results in improved treatment, healing, prevention, or amelioration of a disease, disorder, or side effect, or a decrease in the rate of advancement of a disease or disorder. The term also includes within its scope amounts effective to enhance normal physiological function.

As is well known in the art, tumors are frequently metastatic, in that a first (primary) locus of tumor growth spreads to one or more anatomically separate sites. As used herein, reference to "a tumor" or "a cancer" in a subject includes not only the primary tumor, but metastatic tumor growth as well.

The present invention provides methods for the treatment of several conditions or diseases, all of which comprise the step of administering a compound of formula (I) or a salt or solvate thereof. As used herein, the term "treatment" refers to alleviating the specified condition, eliminating or reducing the symptoms of the condition, slowing or eliminating the progression of the condition and preventing or delaying the initial occurrence of the condition in a subject, or reoccurrance of the condition in a previously afflicted subject.

As used herein, the term "solvate" refers to a complex of variable stoichiometry formed by a solute (in this invention, compounds of formula (I) or a salt thereof) and a solvent. Such solvents for the purpose of the invention may not interfere with the biological activity of the solute. Examples of suitable solvents include, but are not limited to, water, methanol, ethanol and acetic acid. Preferably the solvent used is a pharmaceutically acceptable solvent. Examples of suitable pharmaceutically acceptable solvents include, without limitation, water, ethanol and acetic acid. Most preferably the solvent used is water.

The methods of cancer treatment disclosed herein include administering a compound of formula (1): 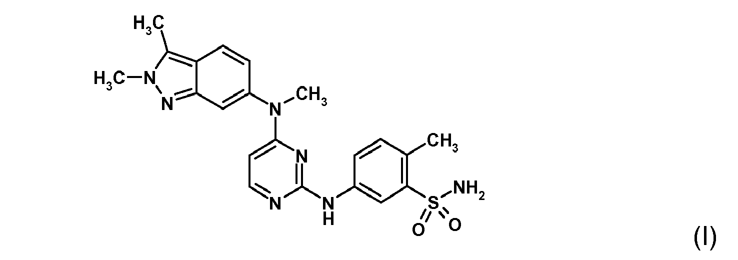 or a salt or solvate thereof.

In one embodiment, the salt of the compound of formula (I) is a hydrochloride salt. In a preferred embodiment, the salt of the compound of formula (I) is a monohydrochloride salt as illustrated by formula (I'). The monohydrochloride salt of the compound of formula (I) has the chemical name 5-[[4-[(2,3-Dimethyl-2H-indazol-6-yl)methylamino]-2-pyrimidinyl]amino]-2-methylbenzenesulfonamide monohydrochloride.

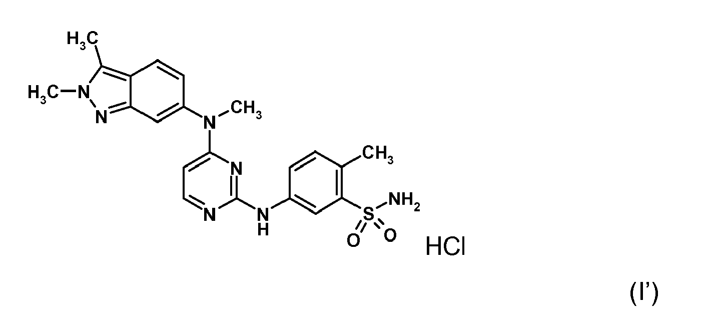

In another preferred embodiment, the the salt of the compound of formula (I) is a monohydrochloride monohydrate solvate of the compound of formula (I). The monohydrochloride monohydrate solvate of the compound of formula (I) has the chemical name 5-({4-[(2,3-dimethyl-2H-indazol-6-yl)methylamino]-2-pyrimidinyl}amino)-2-methylbenzenesulfonamide monohydrochloride monohydrate, as illustrated in formula (1").

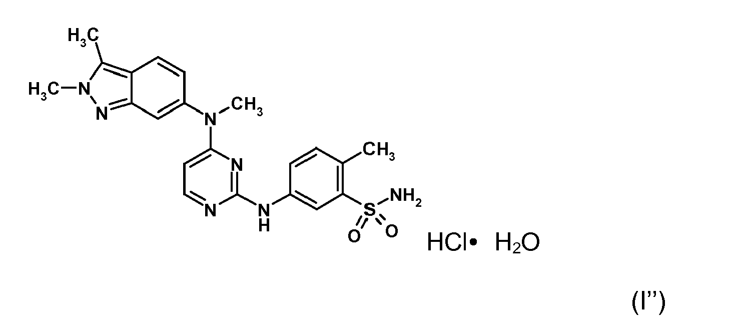

The free base, salts and solvates of the compound of formula (I) may be prepared, for example, according to the procedures of International Patent Application No. PCT/US01/49367 filed December 19, 2001, and published as WO 02/059110 on August 1, 2002, and International Patent Application No. PCT/US03/19211 filed June 17, 2003, and published as WO 03/106416 on December 24, 2003.

Typically, the salts of the present invention are pharmaceutically acceptable salts. Salts encompassed within the term "pharmaceutically acceptable salts" refer to non-toxic salts of the compounds of this invention. Salts of the compounds of the present invention may comprise acid addition salts derived from a nitrogen on a substituent in a compound of the present invention. Representative salts include the following salts: acetate, benzenesulfonate, benzoate, bicarbonate, bisulfate, bitartrate, borate, bromide, calcium edetate, camsylate, carbonate, chloride, clavulanate, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isethionate, lactate, lactobionate, laurate, malate, maleate, mandelate, mesylate, methylbromide, methylnitrate, methylsulfate, monopotassium maleate, mucate, napsylate, nitrate, N-methylglucamine, oxalate, pamoate (embonate), palmitate, pantothenate, phosphate/diphosphate, polygalacturonate, potassium, salicylate, sodium, stearate, subacetate, succinate, tannate, tartrate, teoclate, tosylate, triethiodide, trimethylammonium and valerate. Other salts, which are not pharmaceutically acceptable, may be useful in the preparation of compounds of this invention and these form a further aspect of the invention.

While it is possible that, for use in the cancer treatment methods of the present invention, a compound of formula (I) as well as a salt or solvate thereof, may be administered as the raw chemical, it is possible to present the active ingredient as a pharmaceutical composition. Accordingly, the invention further provides pharmaceutical compositions, which may be administered in the cancer treatment methods of the present invention. The pharmaceutical compositions include a compound of formula (I) and a salt or solvate thereof, and one or more pharmaceutically acceptable carriers, diluents, or excipients. The carrier(s), diluent(s) or excipient(s) must be acceptable in the sense of being compatible with the other ingredients of the formulation and not deleterious to the recipient thereof.

Pharmaceutical formulations may be presented in unit dose forms containing a predetermined amount of active ingredient per unit dose. Such a unit may contain, for example, 0.5mg to 1g, preferably 1mg to 500mg of a compound of formula (I), depending on the condition being treated, the route of administration and the age, weight and condition of the patient, or pharmaceutical formulations may be presented in unit dose forms containing a predetermined amount of active ingredient per unit dose. Preferred unit dosage formulations are those containing a daily dose or sub-dose, as herein above recited, or an appropriate fraction thereof, of an active ingredient. Furthermore, such pharmaceutical formulations may be prepared by any of the methods well known in the pharmacy art.

The compound of formula (I) may be administered by any appropriate route. Suitable routes include oral, rectal, nasal, topical (including buccal and sublingual), vaginal, and parenteral (including subcutaneous, intramuscular, intraveneous, intradermal, intrathecal, and epidural). It will be appreciated that the preferred route may vary with, for example, the condition of the recipient.

The method of the present invention may also be employed with other therapeutic methods of cancer treatment. In particular, in anti-neoplastic therapy, combination therapy with other chemotherapeutic, hormonal, antibody agents as well as surgical and/or radiation treatments other than those mentioned above are envisaged. Anti-neoplastic therapies are described for instance in International Application No. PCT US 02/01130, filed January 14, 2002, published as WO 02/056912 on July 25, 2002, which is incorporated by reference herein to the extent that it relates to anti-neoplastic therapies. Combination therapies according to the present invention thus include the administration of at least one compound of formula (I) as well as optional use of other therapeutic agents including other anti-neoplastic agents. Such combination of agents may be administered together or separately and, when administered separately this may occur simultaneously or sequentially in any order, both close and remote in time. The amounts of the compound of formula (I) and the other pharmaceutically active agent(s) and the relative timings of administration will be selected in order to achieve the desired combined therapeutic effect.

Pharmaceutical formulations adapted for oral administration may be presented as discrete units such as capsules or tablets; powders or granules; solutions or suspensions in aqueous or non-aqueous liquids; edible foams or whips; or oil-in-water liquid emulsions or water-in-oil liquid emulsions.

For instance, for oral administration in the form of a tablet or capsule, the active drug component can be combined with an oral, non-toxic pharmaceutically acceptable inert carrier such as ethanol, glycerol, water and the like. Powders are prepared by comminuting the compound to a suitable fine size and mixing with a similarly comminuted pharmaceutical carrier such as an edible carbohydrate, as, for example, starch or mannitol. Flavoring, preservative, dispersing and coloring agent can also be present.

Capsules can be made by preparing a powder mixture as described above, and filling formed gelatin sheaths. Glidants and lubricants such as colloidal silica, talc, magnesium stearate, calcium stearate or solid polyethylene glycol can be added to the powder mixture before the filling operation. A disintegrating or solubilizing agent such as agar-agar, calcium carbonate or sodium carbonate can also be added to improve the availability of the medicament when the capsule is ingested.

Moreover, when desired or necessary, suitable binders, lubricants, disintegrating agents and coloring agents can also be incorporated into the mixture. Suitable binders include starch, gelatin, natural sugars such as glucose or beta-lactose, corn sweeteners, natural and synthetic gums such as acacia, tragacanth or sodium alginate, carboxymethylcellulose, polyethylene glycol, waxes and the like. Lubricants used in these dosage forms include sodium oleate, sodium stearate, magnesium stearate, sodium benzoate, sodium acetate, sodium chloride and the like. Disintegrators include, without limitation, starch, methyl cellulose, agar, bentonite, xanthan gum and the like. Tablets are formulated, for example, by preparing a powder mixture, granulating or slugging, adding a lubricant and disintegrant and pressing into tablets. A powder mixture is prepared by mixing the compound, suitably comminuted, with a diluent or base as described above, and optionally, with a binder such as carboxymethylcellulose, an aliginate, gelatin, or polyvinyl pyrrolidone, a solution retardant such as paraffin, a resorption accelerator such as a quaternary salt and/or an absorption agent such as bentonite, kaolin or dicalcium phosphate. The powder mixture can be granulated by wetting with a binder such as syrup, starch paste, acadia mucilage or solutions of cellulosic or polymeric materials and forcing through a screen. As an alternative to granulating, the powder mixture can be run through the tablet machine and the result is imperfectly formed slugs broken into granules. The granules can be lubricated to prevent sticking to the tablet forming dies by means of the addition of stearic acid, a stearate salt, talc or mineral oil. The lubricated mixture is then compressed into tablets. The compounds of the present invention can also be combined with free flowing inert carrier and compressed into tablets directly without going through the granulating or slugging steps. A clear or opaque protective coating consisting of a sealing coat of shellac, a coating of sugar or polymeric material and a polish coating of wax can be provided. Dyestuffs can be added to these coatings to distinguish different unit dosages.

Oral fluids such as solution, syrups and elixirs can be prepared in dosage unit form so that a given quantity contains a predetermined amount of the compound. Syrups can be prepared by dissolving the compound in a suitably flavored aqueous solution, while elixirs are prepared through the use of a non-toxic alcoholic vehicle. Suspensions can be formulated by dispersing the compound in a non-toxic vehicle. Solubilizers and emulsifiers such as ethoxylated isostearyl alcohols and polyoxy ethylene sorbitol ethers, preservatives, flavor additive such as peppermint oil or natural sweeteners or saccharin or other artificial sweeteners, and the like can also be added.

Where appropriate, dosage unit formulations for oral administration can be microencapsulated. The formulation can also be prepared to prolong or sustain the release as for example by coating or embedding particulate material in polymers, wax or the like.

The agents for use according to the present invention can also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

Agents for use according to the present invention may also be delivered by the use of monoclonal antibodies as individual carriers to which the compound molecules are coupled. The compounds may also be coupled with soluble polymers as targetable drug carriers. Such polymers can include polyvinylpyrrolidone, pyran copolymer, polyhydroxypropylmethacrylamide-phenol, polyhydroxyethylaspartamidephenol, or polyethyleneoxidepolylysine substituted with palmitoyl residues. Furthermore, the compounds may be coupled to a class of biodegradable polymers useful in achieving controlled release of a drug, for example, polylactic acid, polepsilon caprolactone, polyhydroxy butyric acid, polyorthoesters, polyacetals, polydihydropyrans, polycyanoacrylates and cross-linked or amphipathic block copolymers of hydrogels.

Pharmaceutical formulations adapted for transdermal administration may be presented as discrete patches intended to remain in intimate contact with the epidermis of the recipient for a prolonged period of time. For example, the active ingredient may be delivered from the patch by iontophoresis as generally described in Pharmaceutical Research, 3(6):318 (1986).

Pharmaceutical formulations adapted for topical administration may be formulated as ointments, creams, suspensions, lotions, powders, solutions, pastes, gels, sprays, aerosols or oils.

For treatments of the eye or other external tissues, for example mouth and skin, the formulations are preferably applied as a topical ointment or cream. When formulated in an ointment, the active ingredient may be employed with either a paraffinic or a water-miscible ointment base. Alternatively, the active ingredient may be formulated in a cream with an oil-in-water cream base or a water-in-oil base.

Pharmaceutical formulations adapted for topical administrations to the eye include eye drops wherein the active ingredient is dissolved or suspended in a suitable carrier, especially an aqueous solvent.

Pharmaceutical formulations adapted for topical administration in the mouth include lozenges, pastilles and mouth washes.

Pharmaceutical formulations adapted for rectal administration may be presented as suppositories or as enemas.

Pharmaceutical formulations adapted for nasal administration wherein the carrier is a solid include a coarse powder having a particle size for example in the range 20 to 500 microns which is administered in the manner in which snuff is taken, i.e. by rapid inhalation through the nasal passage from a container of the powder held close up to the nose. Suitable formulations wherein the carrier is a liquid, for administration as a nasal spray or as nasal drops, include aqueous or oil solutions of the active ingredient.

Pharmaceutical formulations adapted for administration by inhalation include fine particle dusts or mists that may be generated by means of various types of metered dose pressurised aerosols, nebulizers or insufflators.

Pharmaceutical formulations adapted for vaginal administration may be presented as pessaries, tampons, creams, gels, pastes, foams or spray formulations.

Pharmaceutical formulations adapted for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets.

It should be understood that in addition to the ingredients particularly mentioned above, the formulations may include other agents conventional in the art having regard to the type of formulation in question, for example those suitable for oral administration may include flavoring agents.

As indicated, a specific compound of formula (I) is administered to a mammal. Typically, the amount of one of the administered agents of the present invention will depend upon a number of factors including, for example, the age and weight of the mammal, the precise condition requiring treatment, the severity of the condition, the nature of the formulation, and the route of administration. Ultimately, the amount will be at the discretion of the attendant physician or veterinarian.

Typically, the compound of formula (I) will be given in the range of 0.1 to 100 mg/kg body weight of recipient (mammal) per day and more usually in the range of 1 to 30 mg/kg body weight per day.

As recited above the present invention includes cancer treatment methods through administration of 5-[[4-[(2,3-Dimethyl-2H-indazol-6-yl)methylamino]-2-pyrimidinyl]amino]-2-methylbenzenesulfonamide or a salt or solvate thereof.

The methods of the present invention are useful for treating many types of susceptible cancers, including but not limited to the following: brain cancer, including glioblastoma multiforme (GBM); neuroendocrine cancer; prostate cancer; myeloma; breast cancer; lung cancer, including non-small cell and mesothelioma; ovarian cancer; kidney cancer, including renal cell carcinoma; gallbladder cancer; liver cancer, including hepatocellular and cholangio; cervical cancer; bladder cancer; head and neck cancer; gastric cancer, including esophageal, stomach, and intestinal; colorectal cancer; skin cancer; smooth muscle cancer; bone cancer; sarcoma; pancreatic cancer; and thyroid cancer.

In one embodiment of the present invention, the treatment may be a first line therapy. In another embodiment, the treatment may be an adjunctive therapy. In a further embodiment, the treatment may be an adjuvant therapy.

In one embodiment of the present invention, there is provided a method of treating a susceptible cancer in a mammal, wherein the susceptible cancer is susceptible to VEGF-R inhibition.

The kidneys are a pair of bean-shaped organs located on each side of the spine that filter blood and eliminate waste in the urine through a complex system of filtration tubules. All of the blood in the body passes through the kidneys approximately 20 times an hour. Renal cell carcinoma (RCC) is an uncommon form of cancer that is most often characterized by the presence of cancer cells in the lining of the kidney's filtration tubules. Cancer that has spread outside the kidney to several and/or distant sites in the body is referred to as metastatic RCC. VEGF has been shown to play a critical role in the biology of RCC. (See, for example, Rini, B.I., The Oncologist 10(3):191-7 (2005)).

In another embodiment of the present invention, there is provided a method of treating a susceptible cancer in a mammal, wherein said cancer is renal cell cancer, comprising: administering to the mammal a compound of formula (I) or a salt or solvate thereof. In another embodiment, the method comprises administering a compound of formula (I'). In another embodiment, the method comprises administering a compound of formula (I").

In another embodiment of the present invention, there is provided a method of treating a susceptible cancer in a mammal, wherein said cancer is renal cell cancer, comprising: administering to the mammal a compound of formula (I) or a salt or solvate thereof and at least one anti-neoplastic therapy. In another embodiment, the method comprises administering a compound of formula (I') and at least one anti-neoplastic therapy. In another embodiment, the method comprises administering a compound of formula (I") and at least one anti-neoplastic therapy.

Melanoma is a very serious form of skin cancer. It begins in melanocytes-cells that make the skin pigment called melanin. Although melanoma accounts for only about 4% of all skin cancer cases, it causes most skin cancer-related deaths. VEGF has been shown to play an important role in the biology of melanoma. (See, for example, Redondo, P. et al., Cytokine 12(4):374-8 (2000)).

In another embodiment of the present invention, there is provided a method of treating a susceptible cancer in a mammal, wherein said cancer is melanoma, comprising: administering to the mammal a compound of formula (I) or a salt or solvate thereof. In another embodiment, the method comprises administering a compound of formula (I'). In another embodiment, the method comprises administering a compound of formula (I").

In another embodiment of the present invention, there is provided a method of treating a susceptible cancer in a mammal, wherein said cancer is melanoma, comprising: administering to the mammal a compound of formula (I) or a salt or solvate thereof and at least one anti-neoplastic therapy. In another embodiment, the method comprises administering a compound of formula (I') and at least one anti-neoplastic therapy. In another embodiment, the method comprises administering a compound of formula (1") and at least one anti-neoplastic therapy.

Sarcoma is a general class of uncommon cancers in which the cancer cells arise from or resemble normal cells in the body known as connective tissue. Normal connective tissues include fat, muscle, blood vessels, deep skin tissues, nerves, bones, and cartilage. Cancers of cells which resemble any of these normal tissues are known as sarcomas. Sarcomas are sub-classified based upon the specific type of cell that makes up the cancer. For example, leiomyosarcoma is a malignant tumor that develops from smooth muscle tissue. Chondrosarcoma is a tumor of cells that form cartlidge. Expression levels of VEGF have been shown to be elevated in sarcomas, and can be correlative to the severity of the tumors. (See, for example, Pakos, EE et al., Anticancer Research, 25(5):3591-6 (2005)).

In another embodiment of the present invention, there is provided a method of treating a susceptible cancer in a mammal, wherein said cancer is sarcoma, comprising: administering to the mammal a compound of formula (I) or a salt or solvate thereof. In another embodiment, the method comprises administering a compound of formula (I'). In another embodiment, the method comprises administering a compound of formula (I").

In another embodiment of the present invention, there is provided a method of treating a susceptible cancer in a mammal, wherein said cancer is sarcoma, comprising: administering to the mammal a compound of formula (I) or a salt or solvate thereof and at least one anti-neoplastic therapy. In another embodiment, the method comprises administering a compound of formula (I') and at least one anti-neoplastic therapy. In another embodiment, the method comprises administering a compound of formula (I") and at least one anti-neoplastic therapy.

Lung cancer is the uncontrolled growth of abnormal cells in one or both of the lungs. While normal lung tissue cells reproduce and develop into healthy lung tissue, these abnormal cells reproduce rapidly and never grow into normal lung tissue. Lumps of cancer cells (tumors) then form and disrupt the lung, making it difficult to function properly.

More than 87% of lung cancers are smoking related. However, not all smokers develop lung cancer. Quitting smoking reduces an individual's risk significantly, although former smokers remain at greater risk for lung cancer than people who never smoked. Exposure to other carcinogens such as asbestos and radon gas also increases an individual's risk, especially when combined with cigarette or cigar smoking. For example, non small cell lung cancer is typically associated with smoking or radon gas exposure. However, most patients with mesothelioma, a rare form of cancer where malignant cells are found in the sac lining the chest (the pleura), the lining of the abdominal cavity (the peritoneum) or the lining around the heart (the pericardium), have been exposed at some time in their lives to asbestos in the workplace or at home. VEGF has been shown to play an important roll in the biology of lung cancers. (See, for example, Huang, C et al., 92(7):1231-9 (2005); and Kumar, P et al., 49 Suppl 1:S53-60 (2005)).

In another embodiment of the present invention, there is provided a method of treating a susceptible cancer in a mammal, wherein said cancer is lung cancer, comprising: administering to the mammal a compound of formula (I) or a salt or solvate thereof. In another embodiment, the method comprises administering a compound of formula (I'). In another embodiment, the method comprises administering a compound of formula (I").

In another embodiment of the present invention, there is provided a method of treating a susceptible cancer in a mammal, wherein said cancer is lung cancer, comprising: administering to the mammal a compound of formula (I) or a salt or solvate thereof and at least one anti-neoplastic therapy. In another embodiment, the method comprises administering a compound of formula (I') and at least one anti-neoplastic therapy. In another embodiment, the method comprises administering a compound of formula (1") and at least one anti-neoplastic therapy.

Stomach cancer, also called gastric cancer, is a cancer that begins in the lining of the stomach. Though the incidence of stomach cancer in the United States has declined, it is still a prominent cause of cancer mortality, especially in developing countries. It occurs most frequently in men over the age of 40, and often presents as later stage disease, since the early stage signs and symptoms frequently go unnoticed. VEGF has been shown to be an important therapeutic target for treating gastric cancers. (See, for example, Whisenant, J et al., Curr Treat Options Oncol. 6(5):411-21 (2005)).

In another embodiment of the present invention, there is provided a method of treating a susceptible cancer in a mammal, wherein said cancer is gastric cancer, comprising: administering to the mammal a compound of formula (I) or a salt or solvate thereof. In another embodiment, the method comprises administering a compound of formula (I'). In another embodiment, the method comprises administering a compound of formula (I").

In another embodiment of the present invention, there is provided a method of treating a susceptible cancer in a mammal, wherein said cancer is gastric cancer, comprising: administering to the mammal a compound of formula (I) or a salt or solvate thereof and at least one anti-neoplastic therapy. In another embodiment, the method comprises administering a compound of formula (I') and at least one anti-neoplastic therapy. In another embodiment, the method comprises administering a compound of formula (I") and at least one anti-neoplastic therapy.

Cancer of the colon or rectum is called colorectal cancer. The cancer occurs when tumors grow in the lining of the large intestine, or large bowel. Occurrence rates are similar between men and women, and risk for the cancer increases after the age of 50. Currently colorectal cancer is the fourth most common cancer in the United States. The VEGF pathway has been shown to be an important target for colorectal cancer therapy. (See, for example, Rhee, J et al., Expert Opin. Pharmacother., 6(10):1701-1711 (2005)).

In another embodiment of the present invention, there is provided a method of treating a susceptible cancer in a mammal, wherein said cancer is colorectal cancer, comprising: administering to the mammal a compound of formula (I) or a salt or solvate thereof. In another embodiment, the method comprises administering a compound of formula (I'). In another embodiment, the method comprises administering a compound of formula (I").

In another embodiment of the present invention, there is provided a method of treating a susceptible cancer in a mammal, wherein said cancer is colorectal cancer, comprising: administering to the mammal a compound of formula (I) or a salt or solvate thereof and at least one anti-neoplastic therapy. In another embodiment, the method comprises administering a compound of formula (I') and at least one anti-neoplastic therapy. In another embodiment, the method comprises administering a compound of formula (1") and at least one anti-neoplastic therapy.

Neuroendocrine tumor refers to the type of cell that a tumor grows from rather than where that tumor is located. Neuroendocrine cells produce hormones or regulatory proteins, and so tumors of these cells usually have symptoms that are related to the specific hormones that they produce. Neuroendocrine cells have roles both in the endocrine system and the nervous system. They produce and secrete a variety of regulatory hormones, or neuropeptides, which include neurotransmitters and growth factors. When these cells become cancerous, they grow and overproduce their specific neuropeptide. Neuroendocrine tumors are generally rare. For example, one type of neuroendocrine tumor is a carcinoid tumor. This type of tumor can occur in the intestinal tract, appendix, rectum, bronchial tubes, or ovary. Most carcinoid tumors secrete serotonin. When the blood concentration of this hormone is high enough, it causes carcinoid syndrome. This syndrome refers to a variety of symptoms that are caused by the excessive amount of hormone secreted rather than the tumor itself. VEGF expression has been observed in neuroendocrine tumors. (See, for example, Tangjitgamol, S et al., Int. J. Gynecol. Cancer, 15(4):646-56 (2005)).

In another embodiment of the present invention, there is provided a method of treating a susceptible cancer in a mammal, wherein said cancer is neuroendocrine cancer, comprising: administering to the mammal a compound of formula (I) or a salt or solvate thereof. In another embodiment, the method comprises administering a compound of formula (I'). In another embodiment, the method comprises administering a compound of formula (I").

In another embodiment of the present invention, there is provided a method of treating a susceptible cancer in a mammal, wherein said cancer is neuroendocrine cancer, comprising: administering to the mammal a compound of formula (I) or a salt or solvate thereof and at least one anti-neoplastic therapy. In another embodiment, the method comprises administering a compound of formula (I') and at least one anti-neoplastic therapy. In another embodiment, the method comprises administering a compound of formula (1") and at least one anti-neoplastic therapy.

Thyroid cancer is the uncontrolled growth of abnormal cells in the thyroid gland. The thyroid gland is shaped like a butterfly and is located under the Adam's apple in the front of the neck. The thyroid has two types of cells that produce two hormones that help to regulate body functions. Follicular cells in the thyroid produce a hormone called thyroxine, or T-4, which controls the body's rate of metabolism. C-cells, also called parafollicular cells, produce calcitonin, a hormone that helps to regulate the level of calcium in the blood. Thyroid cancer is rare, accounting for only about 1.5% of all types of cancer, and the illness is more common in women than in men. VEGF has been shown to play an important role in thyroid cancers. (See, for example, Vieira, JM et al., Eur. J. Endocrinol., 153(5):701-9 (2005)).

In another embodiment of the present invention, there is provided a method of treating a susceptible cancer in a mammal, wherein said cancer is thyroid cancer, comprising: administering to the mammal a compound of formula (I) or a salt or solvate thereof. In another embodiment, the method comprises administering a compound of formula (I'). In another embodiment, the method comprises administering a compound of formula (I").

In another embodiment of the present invention, there is provided a method of treating a susceptible cancer in a mammal, wherein said cancer is thyroid cancer, comprising: administering to the mammal a compound of formula (I) or a salt or solvate thereof and at least one anti-neoplastic therapy. In another embodiment, the method comprises administering a compound of formula (I') and at least one anti-neoplastic therapy. In another embodiment, the method comprises administering a compound of formula (I") and at least one anti-neoplastic therapy.

Breast cancer is a type of cancer where cells in the breast tissue divide and grow without the normal control. About 80 percent of breast cancers originate in the mammary ducts, while about 20 percent arise in the lobules. Cancerous tumors in the breast usually grow very slowly so that by the time one is large enough to be felt as a lump, it may have been growing for as long as ten years. Though breast cancer most often occurs in women, men make up approximately one percent of those diagnosed with breast cancer. VEGF has been associated with breast cancer, and its expression has been suggested to be a prognostic indicator. (See, for example, Rhee, J et al., Expert Opin. Pharmacother., 6(10):1701-1711 (2005)).

In another embodiment of the present invention, there is provided a method of treating a susceptible cancer in a mammal, wherein said cancer is breast cancer, comprising: administering to the mammal a compound of formula (I) or a salt or solvate thereof. In another embodiment, the method comprises administering a compound of formula (I'). In another embodiment, the method comprises administering a compound of formula (I").

In another embodiment of the present invention, there is provided a method of treating a susceptible cancer in a mammal, wherein said cancer is breast cancer, comprising: administering to the mammal a compound of formula (I) or a salt or solvate thereof and at least one anti-neoplastic therapy. In another embodiment, the method comprises administering a compound of formula (I') and at least one anti-neoplastic therapy. In another embodiment, the method comprises administering a compound of formula (I") and at least one anti-neoplastic therapy.

Head and neck cancer is the term given to a variety of malignant tumors that develop in the oral cavity (mouth), pharynx (throat), paranasal sinuses (small hollow spaces around the nose lined with cells that secrete mucus), nasal cavity (airway just behind the nose), larynx ("Adam's apple" or voice box), and salivary glands (parotid, submanidular, sublingual glands that secrete saliva). Some also include skin tumors of the face and neck and tumors of the cervical lymph nodes. Elevated levels of VEGF expression have been found in head and neck cancers. See, for example, Worden, B et al., Cancer Res., 65(16):7071-81 (2005)).

In another embodiment of the present invention, there is provided a method of treating a susceptible cancer in a mammal, wherein said cancer is head and neck cancer, comprising: administering to the mammal a compound of formula (I) or a salt or solvate thereof. In another embodiment, the method comprises administering a compound of formula (I'). In another embodiment, the method comprises administering a compound of formula (I").

In another embodiment of the present invention, there is provided a method of treating a susceptible cancer in a mammal, wherein said cancer is head and neck cancer, comprising: administering to the mammal a compound of formula (I) or a salt or solvate thereof and at least one anti-neoplastic therapy. In another embodiment, the method comprises administering a compound of formula (I') and at least one anti-neoplastic therapy. In another embodiment, the method comprises administering a compound of formula (1") and at least one anti-neoplastic therapy.

Primary brain cancers, such as glioblastoma multiforme (GBM) and anaplastic astrocytoma (AA), are broadly classified as central nervous system cancers. The most common and clinically aggressive brain cancer is GBM. It grows as an irregular mass in the white matter and infiltrates the surrounding parenchyma along the white matter tracts. GBM is characteristically grossly hemorrhagic and necrotic. Despite improvements in therapy, the prognosis remains grave. The median survival for patients with newly diagnosed GBM is approximately 12 months. Relapse occurs in approximately 80% of patients within 6-12 months. Median survival from time of relapse is approximately 6 months. VEGF expression has been observed in brain cancers. (See, for example, Maia, AC Jr. et al., AJNR Am J Neuroradiol., 26(4):777-83 (2005)).

In another embodiment of the present invention, there is provided a method of treating a susceptible cancer in a mammal, wherein said cancer is brain cancer, comprising: administering to the mammal a compound of formula (I) or a salt or solvate thereof. In another embodiment, the method comprises administering a compound of formula (I'). In another embodiment, the method comprises administering a compound of formula (I").

In another embodiment of the present invention, there is provided a method of treating a susceptible cancer in a mammal, wherein said cancer is brain cancer, comprising: administering to the mammal a compound of formula (I) or a salt or solvate thereof and at least one anti-neoplastic therapy. In another embodiment, the method comprises administering a compound of formula (I') and at least one anti-neoplastic therapy. In another embodiment, the method comprises administering a compound of formula (I") and at least one anti-neoplastic therapy.

Cervical cancer is cancer of the uterine cervix, the portion of the uterus attached to the top of the vagina. Ninety percent of cervical cancers arise from the flattened or "squamous" cells covering the cervix. Most of the remaining 10% arise from the glandular, mucus-secreting cells of the cervical canal leading into the uterus. VEGF expression has been associated with cervical cancers at various stages. (See, for example, Mathur, SP et al., Gynecol. Oncol., 98(3):467-83 (2005)).

In another embodiment of the present invention, there is provided a method of treating a susceptible cancer in a mammal, wherein said cancer is cervical cancer, comprising: administering to the mammal a compound of formula (I) or a salt or solvate thereof. In another embodiment, the method comprises administering a compound of formula (I'). In another embodiment, the method comprises administering a compound of formula (I").

In another embodiment of the present invention, there is provided a method of treating a susceptible cancer in a mammal, wherein said cancer is cervical cancer, comprising: administering to the mammal a compound of formula (I) or a salt or solvate thereof and at least one anti-neoplastic therapy. In another embodiment, the method comprises administering a compound of formula (I') and at least one anti-neoplastic therapy. In another embodiment, the method comprises administering a compound of formula (I") and at least one anti-neoplastic therapy.

Cancer of the bladder is the fourth most common malignancy among males and the tenth most common malignancy among females. Each year in the United States, over 50,000 people develop bladder cancer, of whom more than 12,000 ultimately will die of this disease.

Bladder cancer tends to occur most commonly in individuals over 60 years of age. Cigarette smoking and exposure to certain industrially used chemicals (derivatives of compounds called arylamines) are strongly associated with the development of bladder cancer. The vast majority (approximately 90%) of these cancers originate in the lining cells of the bladder, known as urothelium or transitional epithelium. Elevated VEGF expression has been observed in patients with bladder cancer. (See, for example, Beecken, WD et al., J. Cell. Mol. Med., 9(3):655-61 (2005)).

In another embodiment of the present invention, there is provided a method of treating a susceptible cancer in a mammal, wherein said cancer is bladder cancer, comprising: administering to the mammal a compound of formula (I) or a salt or solvate thereof. In another embodiment, the method comprises administering a compound of formula (I'). In another embodiment, the method comprises administering a compound of formula (I").

In another embodiment of the present invention, there is provided a method of treating a susceptible cancer in a mammal, wherein said cancer is bladder cancer, comprising: administering to the mammal a compound of formula (I) or a salt or solvate thereof and at least one anti-neoplastic therapy. In another embodiment, the method comprises administering a compound of formula (I') and at least one anti-neoplastic therapy. In another embodiment, the method comprises administering a compound of formula (I") and at least one anti-neoplastic therapy.

Esophageal cancer is a malignant tumor of the esophagus, the muscular tube that transports food from the mouth to the stomach. Esophageal cancer is relatively uncommon in the United States, and occurs most often in men over 50 years old. It affects less than 5 in 100,000 people. There are two main types of esophageal cancer, squamous cell carcinoma and adenocarcinoma. These two types are distinguished by the way they look under the microscope.

Squamous cell cancer is associated with smoking and alcohol consumption. The incidence of this disease in the United States has remained relatively constant, while the incidence of adenocarcinoma of the esophagus has risen dramatically. Barrett's esophagus, a complication of gastroesophageal reflux disease (GERD), is a risk factor for the development of adenocarcinoma of the esophagus. Risk factors for adenocarcinoma of the esophagus include male gender, obesity, western diet, and smoking. VEGF has been shown to play a role in esophageal cancers. (See, for example, lshikawa, M et al., Hepatogastroenterology, 51 (59):1319-22 (2004)).

In another embodiment of the present invention, there is provided a method of treating a susceptible cancer in a mammal, wherein said cancer is esophageal cancer, comprising: administering to the mammal a compound of formula (I) or a salt or solvate thereof. In another embodiment, the method comprises administering a compound of formula (I'). In another embodiment, the method comprises administering a compound of formula (I").

In another embodiment of the present invention, there is provided a method of treating a susceptible cancer in a mammal, wherein said cancer is esophageal cancer, comprising: administering to the mammal a compound of formula (I) or a salt or solvate thereof and at least one anti-neoplastic therapy. In another embodiment, the method comprises administering a compound of formula (I') and at least one anti-neoplastic therapy. In another embodiment, the method comprises administering a compound of formula (1") and at least one anti-neoplastic therapy.

Cancer of the pancreas is a disease in which cancer (malignant) cells are found in the tissues of the pancreas. The pancreas is about 6 inches long and is shaped something like a thin pear, wider at one end and narrowing at the other. The pancreas lies behind the stomach, inside a loop formed by part of the small intestine. The broader right end of the pancreas is called the head, the middle section is called the body, and the narrow left end is the tail.

The pancreas has two basic jobs in the body. It produces juices that help digest food, and hormones (such as insulin) that regulate how the body stores and uses food. The area of the pancreas that produces digestive juices is called the exocrine pancreas. About 95% of pancreatic cancers begin in the exocrine pancreas. The hormone-producing area of the pancreas is called the endocrine pancreas. Only about 5% of pancreatic cancers start here, and include islet cell carcinomas. VEGF expression has been demonstrated in pancreatic cancers, and levels of expression may be correlated with prognosis. (See, for example, Rhee, J et al., Expert Opin. Pharmacother., 6(10):1701-1711 (2005)).

In another embodiment of the present invention, there is provided a method of treating a susceptible cancer in a mammal, wherein said cancer is pancreatic cancer, comprising: administering to the mammal a compound of formula (I) or a salt or solvate thereof. In another embodiment, the method comprises administering a compound of formula (I'). In another embodiment, the method comprises administering a compound of formula (I").

In another embodiment of the present invention, there is provided a method of treating a susceptible cancer in a mammal, wherein said cancer is pancreatic cancer, comprising: administering to the mammal a compound of formula (I) or a salt or solvate thereof and at least one anti-neoplastic therapy. In another embodiment, the method comprises administering a compound of formula (I'). In another embodiment, the method comprises administering a compound of formula (I").

There are two main kinds of liver cancer. Heptoma and cholangiocarcinoma. Heptoma is cancer of the hepatocytes. (The main functioning liver cell). Hepatoma is primary liver cancer. Hepatoma usually grows in the liver as a ball-like tumor, invading the normal tissue surrounding it. A history of infection with the hepatitis B virus puts individuals at risk of developing heptoma.

Cancer of the bile duct cells is called cholangiocarcinoma. Cholangiocarcinoma originates in the bile ducts and is often caused by infestation with the liver fluke Clonorchis (a parasite). The cancer grows along the bile ducts in sheets or lines, and is hard to find on X-ray studies.

Liver cancer is much more prevalent in many of the developing countries than in the industrialized world. Its incidence is highest in subSaharan Africa, China, southern Asia, and Japan. Japan is the exception of the industrialized countries. China accounts for about 45% of the world's cases. VEGF expression levels have been shown to be important prognostic markers in liver cancers. (See, for example, Deng-Fu, Y et al., Hepatobiliary Pancreat. Dis. lnt., 4(2):220-6 (2005)).

In another embodiment of the present invention, there is provided a method of treating a susceptible cancer in a mammal, wherein said cancer is liver cancer, comprising: administering to the mammal a compound of formula (I) or a salt or solvate thereof. In another embodiment, the method comprises administering a compound of formula (I'). In another embodiment, the method comprises administering a compound of formula (I").

In another embodiment of the present invention, there is provided a method of treating a susceptible cancer in a mammal, wherein said cancer is liver cancer, comprising: administering to the mammal a compound of formula (I) or a salt or solvate thereof and at least one anti-neoplastic therapy. In another embodiment, the method comprises administering a compound of formula (I'). In another embodiment, the method comprises administering a compound of formula (I").

In the foregoing cancer treatment methods of the present invention the compounds of formulae (I), (I') and (I") are as described above.

The following examples are intended for illustration only and are not intended to limit the scope of the invention in any way.

### EXAMPLES

As used herein the symbols and conventions used in these processes, schemes and examples are consistent with those used in the contemporary scientific literature, for example, the *Journal of the American Chemical Society* or the *Journal of Biological Chemistry.* Standard single-letter or three-letter abbreviations are generally used to designate amino acid residues, which are assumed to be in the L-configuration unless otherwise noted. Unless otherwise noted, all starting materials were obtained from commercial suppliers and used without further purification. Specifically, the following abbreviations may be used in the examples and throughout the specification:

| | |
|---|---|
| g (grams); | mg (milligrams); |
| L (liters); | mL (milliliters); |
| µL (microliters); | psi (pounds per square inch); |
| M (molar); | mM (millimolar); |
| N (Normal) | Kg (kilogram) |
| i. v. (intravenous); | Hz (Hertz); |
| MHz (megahertz); | mol (moles); |
| mmol (millimoles); | RT (room temperature); |
| min (minutes); | h (hours); |
| mp (melting point); | TLC (thin layer chromatography); |
| Tᵣ (retention time); | RP (reverse phase); |
| DCM (dichloromethane); | DCE (dichloroethane); |
| DMF (N,N-dimethylformamide); | HOAc (acetic acid); |
| TMSE (2-(trimethylsilyl)ethyl); | TMS (trimethylsilyl); |
| TIPS (triisopropylsilyl); | TBS (t-butyldimethylsilyl); |
| HPLC (high pressure liquid | chromatography); |
| THF (tetrahydrofuran); | DMSO (dimethylsulfoxide); |
| EtOAc (ethyl acetate); | DME (1,2-dimethoxyethane); |
| EDTA ethylenediaminetetraacetic acid; | |
| FBS fetal bovine serum; | |
| IMDM Iscove's Modified Dulbecco's medium; | |
| PBS phosphate buffered saline; | |
| RPMI Roswell Park Memorial Institute; | |
| RIPA buffer *; | |
| RT room temperature; | |
| QD once a day; | BID twice a day; |
| SD stable disease; | MR mixed response; |
| PR partial remission. | |

| | |
|---|---|
| *150 mM NaCl, 50 mM Tris-HCl, pH 7.5, 0.25% (w/v) -deoxycholate, 1% NP-40, 5 mM sodium orthovanadate, 2 mM sodium fluoride, and a protease inhibitor cocktail. | |

Unless otherwise indicated, all temperatures are expressed in °C (degrees Centigrade). All reactions conducted under an inert atmosphere at room temperature unless otherwise noted.

The following examples describe the syntheses of intermediates particularly useful in the synthesis of compounds of formula (I):

### Intermediate Example 1

### Preparation of 2,3-dimethyl-6-nitro-2H-indazole

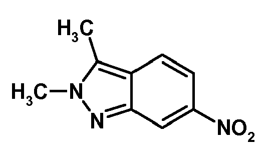

### Procedure 1:

To a stirred solution of 18.5 g (0.11 mol) of 3-methyl-6-nitro-*1H*-indazole in 350 ml acetone, at room temperature, was added 20 g (0.14 mol) of trimethyloxonium tetraflouroborate. After the solution was allowed to stir under argon for 3 hours, the solvent was removed under reduced pressure. To the resulting solid was added saturated aqueous NaHCO₃ (600 mL) and a 4:1 mixture of chloroform-isopropanol (200 ml), the mixture was agitated and the layers were separated. The aqueous phase was washed with additional chloroform: isopropanol (4 x 200 mL) and the combined organic phase was dried (Na₂S0₄). Filtration and removal of solvent gave a tan solid. The solid was washed with ether (200 mL) to afford 2,3-dimethyl-6-nitro-2H-indazole as a yellow solid (15.85 g, 73 %). ¹H NMR (300 MHz, DMSO-d₆)δ 8.51 (s, 1H), 7.94 (d, *J* = 9.1 Hz, 1H), 7.73 (d, *J* = 8.9 Hz, 1H), 4.14 (s, 3H), 2.67 (s, 3H). MS (ES+, m/z) 192 (M+H).

### Procedure 2:

Trimethyl orthoformate (11 mmol, 1.17 g) was added over a 2 min period to a solution of boron trifluoride etherate (12.5 mmol, 1.77 g in methylene chloride (2.0 mL) which had been cooled to -30 °C. The mixture was warmed to 0 °C for 15 min and was then cooled to -70 °C. The nitro indazole (10 mmol, 1.77 g) was slurried in methylene chloride (30 mL) and was added all at once to the cooled mixture. The mixture was stirred at -70 °C for 15 min and at ambient temperature for 17 h. After 17 h the mixture was red and heterogeneous. The reaction mixture was quenched with saturated sodium bicarbonate solution (20 mL) and the organic layer separated. The aqueous layer was extracted with methylene chloride (30 mL). The methylene chloride layers were combined and extracted with water (30 mL). The methylene chloride layer was distilled under reduced pressure until ∼ 10 mL remained. Propanol (10 mL) was added and the remainder of the methylene chloride removed under reduced pressure, resulting in a yellow slurry. The product was isolated by filtration to give 2,3-dimethyl-6-nitro-2H-indazole (65 %, 7mmol, 1.25 g) as a light yellow powder. ¹H NMR (300 MHz, DMSO-d₆) δ 8.51 (s, 1H), 7.94 (d, *J* = 9.1 Hz, 1H), 7.73 (d, *J* = 8.9 Hz, 1H), 4.14 (s, 3H), 2.67 (s, 3H). MS (ES+, m/z) 192 (M+H).

### Procedure 3:

In a 25 ml round bottom flask 3-methyl-6-nitroindazole (7.27 mmol, 1.28 g) was dissolved with stirring in DMSO (4.0 mL) and was treated with concentrated sulfuric acid (7.27 mmol, 0.73 g) to yield a thick slurry. The slurry was treated with dimethyl sulfate (21.1 mmol, 2.66 g). The mixture was heated under nitrogen at 50 °C for 72 h. After 72 h a thick yellow slurry was obtained. The slurry was cooled and was slowly treated with saturated sodium bicarbonate solution (10 mL). The mixture was extracted with methylene chloride (2 x 20 mL). The methylene chloride layers were combined and back extracted with water (20 mL). The methylene chloride layer was treated with propanol (10 mL) and the methylene chloride was removed by distillation under reduced pressure. The solid was isolated by filtration and the yellow solid washed with heptane (5 mL) and air-dried. The 2,3-dimethyl-6-nitro-*2H*-indazole product (70%, 0.97 g) was obtained as a light yellow solid. ¹H NMR (300 MHz, DMSO-d₆) δ 8.51 (s, 1H), 7.94 (d, *J* = 9.1 Hz, 1H), 7.73 (d, *J* = 8.9 Hz, 1H), 4.14 (s, 3H), 2.67 (s, 3H). MS (ES+, m/z) 192 (M+H).

### Procedure 4:

Into a 250 mL 3-necked round bottom flask was placed 3-methyl-6-nitro-1H-indazole sulfuric acid salt (5.0 g, 18.2 mmol) and methylene chloride (25 mL). The mixture was stirred at 25 °C and was treated with DMSO (5 mL). Dimethyl sulfate (6.7 g, 5.0 mL, 53.0 mmol) was added via syringe and the reaction was heated at reflux in a 70 °C bath. After 7 h HPLC analysis showed 9% starting material. At this point heating was stopped and the workup begun. Saturated sodium bicarbonate solution (35 mL) was added to the reaction mixture at RT. The layers were allowed to separate and the aqueous layer was extracted with methylene chloride (25 mL). The methylene chloride layers were combined and washed with water (2 x 25 mL). The methylene chloride layer was distilled under reduced pressure until half the volume was removed. Propanol (25 mL) was added and distillation under reduced pressure was continued until all the methylene chloride had been removed. This yielded a yellow slurry, which was allowed to stir at 25 °C for 1 h. The product was isolated via filtration and the resulting yellow solid was washed with heptane (10 mL). This yielded 2,3-dimethyl-6-nitro-2H-indazole (70%, 2.43 g) as a yellow solid. ¹H NMR (300 MHz, DMSO-d₆) δ 8.51 (s, 1H), 7.94 (d, *J* = 9.1 Hz, 1H), 7.73 (d, *J* = 8.9 Hz, 1H), 4.14 (s, 3H), 2.67 (s, 3H). MS (ES+, m/z) 192 (M+H).

### Intermediate Example 2

### Preparation of 2,3-dimethyl-6-amino-2H-indazole:

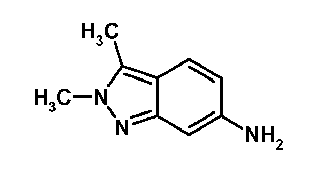

### Procedure 1:

To a stirred solution of 2,3-dimethyl-6-nitro-2H-indazole (1.13 g) in 2-methoxyethyl ether (12 ml), at 0 °C, was added a solution of 4.48 g of tin(ll) chloride in 8.9 ml of concentrated HCI dropwise over 5 min. After the addition was complete, the ice bath was removed and the solution was allowed to stir for an additional 30 min. Approximately 40 ml of diethyl ether was added to reaction, resulting in precipitate formation. The resulting precipitate was isolated by filtration and washed with diethyl ether, and afforded a yellow solid (1.1 g, 95 %), the HCl salt 2,3-dimethyl-*2H*-indazol-6-amine. ¹H NMR (300 MHz, DMSO-d₆) δ 7.77 (d, *J* = 8.9 Hz, 1H), 7.18 (s, 1H), 7.88 (m, 1H), 4.04 (s, 3H), 2.61 (s, 3H). MS (ES+, m/z) 162 (M+H).

### Procedure 2:

A 2-L 3-necked round bottom flask was fitted with nitrogen inlet and outlet and with mechanical stirring. A moderate nitrogen flow was initiated and the reactor was charged with 10 % Pd/C (50% water wet, 6.0 g). Stirring was initiated and the reactor was charged with methanol (750 mL) and the product of Intermediate Example 1 (50 g). Ammonium formate (82.54 g) was dissolved in water (120 mL). The water solution of ammonium formate was added to the reaction solution at an addition rate, which kept the reaction temperature at or between 25 and 30°C. The reaction was allowed to proceed at 25°C. After 6 h the reaction was judged to be finished based on HPLC analysis. The mixture was filtered and the catalyst washed with methanol (50 mL). The methanol layers were combined and the solvent removed under reduced pressure. The residue was dissolved in water (200 mL) and was extracted with methylene chloride (3 x 250 mL). The methylene chloride layers were combined and solvent removed under vacuum to remove approximately half the solvent. Heptane (400 mL) was added and the vacuum distillation continued until approximately 300 mL reaction product slurry remained. The product was isolated by filtration and dried under vacuum at 50°C for 4 h. to yield 2,3-dimethyl-6-amino-2H-indazole as the free base. (40.76 g, 96.7 %). ¹H NMR (300 MHz, DMSO-d₆) δ 7.31 (d, *J* = 8.9 Hz, 1H), 6.45 (d, *J* = 8.9 Hz, 1H), 6.38 (s, 1H), 4.95 (s, br, 2H), 3.85 (s, 3H), 2.44 (s, 3H) MS (ES+, m/z) 162 (M+H).

### Intermediate Example 3

### Preparation of N-(2-chloropyrimidin-4-yl)-2,3-dimethyl-2H-indazol-6-amine

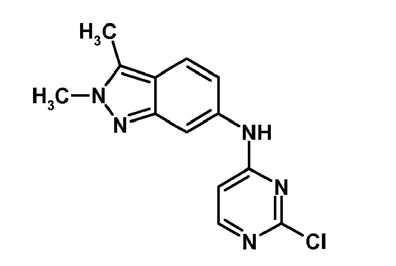

### Procedure 1

To a stirred solution of the product of Intermediate Example 2 (2.97 g, .015 mol) and NaHCO₃(5.05 g, .06 mol) in THF (15 mL) and ethanol (60 mL) was added 2,4-dichloropyrimidine (6.70 g, .045 mol) at rt. After the reaction was stirred for four hours at 85 °C, the suspension was cooled to rt., filtered and washed thoroughly with ethyl acetate. The filtrate was concentrated under reduced pressure, and the resulting solid was triturated with ethyl acetate to yield *N*-(2-chloropyrimidin-4-yl)-2,3-dimethyl-2*H*-indazol-6-amine (89 %, 3.84 g). ¹H NMR (400 MHz, DMSO-d₆) δ 7.28 (d, *J* = 9.0 Hz, 1H), 6.42 (d, *J* = 8.8 Hz, 1H), 6.37 (s, 1H), 5.18 (br s, 1H), 3.84 (s, 3H), 2.43 (s, 3H). MS (ES+, m/z) 274 (M+H).

### Procedure 2

To a 1-L 3-necked flask equipped with air-driven mechanical stirrer, thermometer, and nitrogen inlet/outlet was charged a solution of the product of Intermediate Example 2 (32.89 g, 0.204 mol, 1.0 equiv) in 425 mL (13 volumes) of EtOH/THF (4/1), sodium bicarbonate (51.42 g, 0.612 mol, 3.0 equiv) and then 2,4-dichloropyrimidine (45.59 g, 0.306 mol, 1.5 equiv). The flask contents were heated to 75°C and held at 74 - 76 °C for 6 - 7 hrs. The progress of the reaction was checked by HPLC (the product of Intermediate Example 2 < 2%). The reaction contents were cooled to 20 - 25 °C over 30 min, and kept at 20 ― 25 °C for 30 min. Then the reaction contents were further cooled to 10 - 12°C over 30 min, and kept at that temperature for an additional 10 min. The contents were filtered and filter cake washed with EtOAc (2 x 100 mL, 3.0 volumes), and deionized water (514 mL, 15.6 volumes). The filter cake was then dried in a vacuum oven at 35°C overnight to afford the desired product 44.75 g as a white solid (80.1 %). ¹H NMR (400 MHz, DMSO-d₆) δ 7.28 (d, *J* = 9.0 Hz, 1 H), 6.42 (d, *J* = 8.8 Hz, 1H), 6.37 (s, 1H), 5.18 (br s, 1H), 3.84 (s, 3H), 2.43 (s, 3H). MS (ES+, m/z) 274 (M+H).

### Procedure 3

To a 2 L jacketed reactor was charged with IMS (1000 mL), the product of Intermediate Example 2 (100 g, 0.620 mol, 1 equiv), Sodium Hydrogen Carbonate (107g, 1.27 mol, 2.05 equiv), and 2,4-dichloropyrimidine (101 g, 0.682 mol, 1.1 equiv). The solution was stirred and heated to reflux with a jacket temperature of 85 °C for 8 hours. The resulting slurry was then cooled to 50 °C, and water (500 mL) was added to maintain the temperature between 40 and 50°C. The reaction was then stirred at an internal temperature of 50°C for one hour, and then cooled to 20°C. The solid product was collected by filtration, washed with water (750 mL X 2), and followed by with EtOAc (450 mL X 1 ). After drying at overnight, under vacuum at 60 °C afforded 135 g (80%) of *N*-(2-chloropyrimidin-4-yl)-2,3-dimethyl-2*H*-indazol-6-amine.

### Intermediate Example 4

### Preparation of N-(2-chloropyrimidin-4-yl)-N,2,3-trimethyl-2H-indazol-6-amine

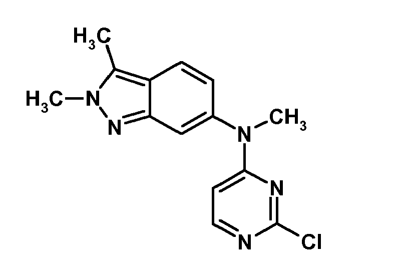

### Procedure 1

To a stirred solution of the product of Intermediate Example 3 (7.37 g) in DMF (50 ml) was added CS₂CO₃ (7.44 g, 2 eqv.) and iodomethane (1.84 ml, 1.1 eqv.) at room temperature. The mixture was stirred at rt overnight. The reaction mixture was then poured into an ice-water bath, and the precipitate was collected via filtration and washed with water. The precipitate was air-dried to afford *N*-(2-chloropyrimidin-4-yl)-N,2,3-trimethyl-2H-indazol-6-amine as an off-white solid (6.43 g, 83%). ¹H NMR (400 MHz, DMSO-d₆) δ 7.94 (d, *J* = 6.0 Hz, 1H), 7.80 (d, *J* = 7.0 Hz, 1H), 7.50 (d, *J* = 1.0 Hz, 1H), 6.88 (m, 1H), 6.24 (d, *J* = 6.2 Hz, 1H), 4.06 (s, 3H), 3.42 (s, 3H), 2.62 (s, 3H). MS (ES+, m/z) 288 (M+H).

### Procedure 2

A 3L 3-necked flask equipped with air-driven mechanical stirrer, thermometer, addition funnel and nitrogen inlet/outlet was charged with DMF (272 mL, 5 volumes) and the product of Intermediate Example 3 (54.4 g, 0.20 mol, 1.0 equiv) with stirring. The reaction mixture was further charged with cesium carbonate (194.5 g, 0.60 mol, 3.0 equiv) while maintaining the reaction temperature between 20 ∼ 25°C. The reaction mixture was stirred at 20 ∼ 25°C for 10 minutes. Iodomethane (45.1 g, 0.32 mol, 1.6 equiv) was charged over ∼ 10 minutes while maintaining the temperature 20 ∼ 30°C. The reaction mixture was stirred at 20 ∼ 30°C (Typically, the reaction is complete in 1 ∼ 2 hours). Deionized H₂O (925 mL, 17 volumes) was added over ∼ 30 minutes while maintaining the temperature at 25 ∼ 40°C. The reaction mixture was stirred at 20 ∼ 25°C for 40 minutes. The product was isolated by filtration and then the filter cake washed with H₂O / DMF (6 : 1, 252 mL, 4.6 volumes). The wet cake was dried under vacuum at 40 ∼ 45°C and *N*-(2-chloropyrimidin-4-yl)-*N*,2,3-trimethyl-2*H-*indazol-6-amine (51.7 g, 90.4%) was isolated as a yellow solid. ¹H NMR (400 MHz, DMSO-d₆) δ 7.94 (d, *J* = 6.0 Hz, 1H), 7.80 (d, *J* = 7.0 Hz, 1H), 7.50 (d, *J* = 1.0 Hz, 1H), 6.88 (m, 1H), 6.24 (d, *J* = 6.2 Hz, 1H), 4.06 (s, 3H), 3.42 (s, 3H), 2.62 (s, 3H). MS (ES+, m/z) 288 (M+H).

### Procedure 3

To a 2 L jacketed reactor was charged with DMF (383 mL), dimethyl carbonate (192 mL), the product of Intermediate Example 3 (115 g, 0.420 mol, 1 equiv) and Potassium Carbonate (174 g, 1.26 mol, 3 equiv). The suspension was stirred and heated to reflux with a jacket temperature of 135°C for 6 hours. The resulting slurry was then cooled to 60°C, and water (1150 mL) was added slowly maintaining the reaction temperature between 50 and 65 °C. The reaction was then cooled down to 20°C and stirred at an internal temperature of 20°C for two hours, and then cooled to 10°C and held overnight after which it was filtered. The solid was washed with water (230 mL X 2) at room temperature, and rinsed with the mixture IMS:Water (1:1) (230 mL X 1). After drying at overnight, under vacuum at 60°C afforded 101 g (83%) of N-(2-chloropyrimidin-4-yl)-N,2,3-trimethyl-2H-indazol-6-amine.

### Example 1: Preparation of pazopanib (5-(f4-[(2,3-dimethyl-2H-indazol-6-yl)(methyl)amino]pyrimidin-2-yl}amino)-2-methylbenzenesulfonamide) and salts and solvates thereof

### Example 1a

### Preparation of 5-({4-[(2,3-dimethyl-2H-indazol-6-yl)(methyl)amino]pyrimidin-2-yl}amino)-2-methylbenzenesulfonamide

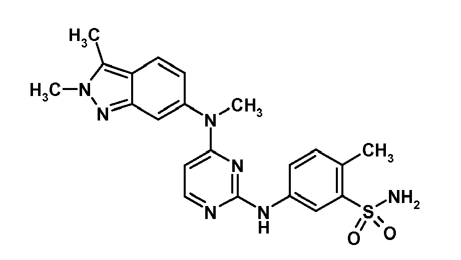

### Procedure 1

To a solution of Intermediate Example 4 (200 mg, 0.695 mmol) and 5-amino-2-methylbenzenesulfonamide (129.4 mg, 0.695 mmol) in isopropanol (6 ml) was added 4 drops of conc. HCl. The mixture was heated to reflux overnight. The mixture was cooled to rt and diluted with ether (6 ml). Precipitate was collected via filtration and washed with ether. The hydrochloride salt of 5-({4-[(2,3-dimethyl-2H-indazol-6-yl)(methyl)amino]-pyrimidin-2-yl)amino)-2-methylbenzenesulfonamide was isolated as an off-white solid. ¹H NMR (400 MHz, d₆DMSO+NaHC0₃) δ 9.50 (br s, 1H), 8.55 (br s, 1H), 7.81 (d, *J* = 6.2 Hz, 1H), 7.75 (d, J = 8.7 Hz, 1H), 7.69 (m, 1H), 7.43 (s, 1H), 7.23 (s, 2H), 7.15 (d, *J* = 8.4 Hz, 1H), 6.86 (m, 1H), 5.74 (d, *J* = 6.1 Hz, 1H), 4.04 (s, 3H), 3.48 (s, 3H), 2.61 (s, 3H), 2.48 (s, 3H). MS (ES+, m/z) 438 (M+H).

### Procedure 2

A 250-mL 3-necked flask equipped with a magnetic stir bar, thermometer, reflux condenser, and nitrogen inlet/outlet was charged with ethanol (60 mL, 10 volumes), the product of Intermediate Example 4 (6.00 g, 20.85 mmol, 1.0 equiv) and 5-amino-2-methylbenzenesulfonamide (4.00 g, 21.48 mmol, 1.03 equiv) with stirring. The reaction mixture was heated to 70 °C. After stirring the reaction mixture at 68 - 72 °C for 3 hrs, 4M HCl in dioxane (0.11 mL, 0.44 mmol, 0.02 equiv) was charged over ca. 2 min. The reaction mixture was stirred at 68 - 72 °C until < 1.5% by area of the starting product of Intermediate Example 4 was remaining by HPLC analysis (Typically, this reaction is complete in > 8 hrs). The reaction mixture was cooled to 20 °C over ca. 30 min and stirred at 20 - 22 °C for 40 min. The product was then isolated by filtration and the filter cake washed with ethanol (20 mL, 3.3 volumes). The wet cake was dried under vacuum at 45 - 50 °C. The monohydrochloride salt of 5-({4-[(2,3-dimethyl-2*H*-ind azol-6-yl)(methyl)amino]-pyrimidin-2-yl}amino)-2-methylbenzenesulfonamide (9.52 g, 96.4%) was isolated as a white solid.¹H NMR (400 MHz, d₆DMSO+NaHCO₃) δ 9.50 (br s, 1H), 8.55 (br s, 1H), 7.81 (d, *J* = 6.2 Hz, 1H), 7.75 (d, *J* = 8.7 Hz, 1H), 7.69 (m, 1H), 7.43 (s, 1H), 7.23 (s, 2H), 7.15 (d, *J* = 8.4 Hz, 1H), 6.86 (m, 1H), 5.74 (d, *J* = 6.1 Hz, 1H), 4.04 (s, 3H), 3.48 (s, 3H), 2.61 (s, 3H), 2.48 (s, 3H). MS (ES+, m/z) 438 (M+H).

### Procedure 3:

To a stirred suspension of the product of Intermediate Example 4 (1.1 g, 3.8 mmol) in 14 mL of MeOH, was added 5-amino-2-methylbenzenesulfonamide (0.78 g, 4.2 mmol, 1.1 equiv) at room temperature. The reaction mixture was heated at reflux for 3 h, then 4 M HCl in 1,4-dioxane (19 µL, 0.076 mmol) was added in one portion. After 4 h, the suspension was cooled to room temperature, and filtered. The resulting solid was washed with 10 mL of MeOH and dried in vacuo to yield 1.3 g (72%) of 5-({4-[(2,3-dimethyl-2H-indazol-6-yl)methylamino]-2-pyrimidinyllamino)-2-methyl benzenesulfonamide monohydrochloride as a white solid. ¹H NMR (DMSO-d6, 400 MHz) δ 10.95 (s, 1H), 8.36 (s, 1H), 7.86 (d, *J* = 8.8 Hz, 2H), 7.64-7.59 (m, 2H), 7.40 (m, 3H), 6.93 (dd, *J* = 8.8, 2.0 Hz, 1H), 5.92 (s, 1H), 4.08 (s, 3H), 3.57 (s, 3H), 2.65 (s, 3H), 2.56 (s, 3H).

### Procedure 4

To a stirred suspension of the product of Intermediate Example 4 (1.1 g, 3.7 mmol) in 10 mL of THF, was added 5-amino-2-methylbenzenesulfonamide (0.70 g, 3.8 mmol, 1.0 equiv) at room temperature. The reaction mixture was heated at reflux for 3 h, then 4 M HCl in 1,4-dioxane (18 µL, 0.072 mmol) was added in one portion. After 5 h, the suspension was cooled to room temperature, and filtered. The resulting solid was washed with 16 mL of THF and dried in the air to yield 1.6 g (92%) of 5-({4-[(2,3-dimethyl-2H-indazol-6-yl)methylamino]-2-pyrimidinyl}amino)-2-methylbenzene sulfonamide monohydrochloride as a light yellow solid.

### Procedure 5

To a stirred suspension of the product of Intermediate Example 4 (1.0 g, 3.6 mmol) in 10 mL of CH₃CN, was added 5-amino-2-methylbenzenesulfonamide (0.70 g, 3.8 mmol, 1.Oequiv) at room temperature. The reaction mixture was heated at reflux for 3 h, then 4 M HCI in 1,4-dioxane (18 µL, 0.076 mmol) was added in one portion. After 20 h, the suspension was cooled to room temperature, and filtered. The resulting solid was washed with 10 mL of CH₃CN and dried in the air to yield 1.3 g (73%) of 5-({4-[(2,3-dimethyl-2H-indazol-6-yl)methylamino]-2-pyrimidinyl}amino)-2-methyl benzenesulfonamide monohydrochloride as an off-white solid.

### Procedure 6

To a 2 L jacketed reactor was charged with MeOH (1005 mL), the product of Intermediate Example 4 (84 g, 0.292 mol, 1 equiv) and 5-amino-2-methylbenzenesulfonamide (60 g, 0.320 mol, 1.1 equiv). The solution was stirred and heated to 50 °C and 4M HCI in Dioxane (1.46 mL, 2 mol%) was added. The solution was then stirred and heated to reflux with a jacket temperature of 85 °C for 10 hours. The resulting slurry was then cooled to 20-25 °C and filtered. The filtered solid was washed with acetonitrile (293 mL X 2) at room temperature. After drying at overnight, under vacuum at 60 °C afforded 116 g (81 %) of 5-({4-[(2,3-dimethyl-2H-indazol-6-yl)methylamino]-2-pyrimidinyl)amino)-2-methyl benzenesulfonamide monohydrochloride.

### Example 1b

### Preparation of 5-({4-[(2,3-dimethlyl-2H-indazol-6-yl)methylamino]-2-pyrimidinyl}amino)-2-methylbenzenesulfonamide monohydrochloride monohydrate.

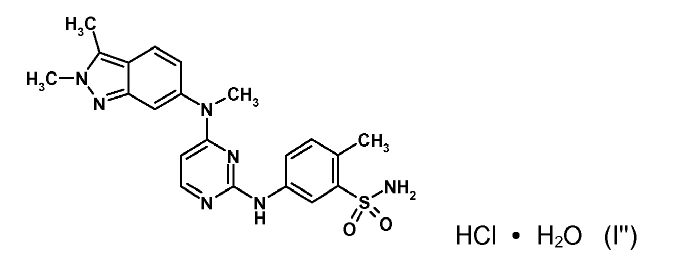

To a round bottom flask, was added 2.6 g of the monohydrochloride salt of Example 1 a, procedure 1, any form. Then added was 39 mL of isopropanol (15 volumes). The mixture was heated to 75 deg C in an oil bath, then 14 mL of 0.05N aqueous HCI (5.4 volumes) was added. The clear solution was cooled to 65 deg C, then seeded with the monohydrate of the monohydrochloride salt of Example 1, procedure 1 (0.05-0.1 wt %). The cloudy solution was stirred at 65 deg C for 60 minutes, then cooled to 0 deg C at ∼0.25-0.5 deg C/min. The resulting white solid was filtered and dried to constant weight under vacuum at RT to give 88% yield of 5 -({4-[(2,3-dimethyl-2H-indazol-6-yl)methylamino]-2-pyrimidinyl}amino)-2-methylbenzene sulfonamide monohydrochloride monohydrate.

### Example 1c

### Preparation of 5-({4-[(2,3-dimethyl-2H-indazol-6-yl)methylamino]-2-pyrimidinyl}amino)-2-methylbenzenesulfonamide monohydrochloride anhydrate.

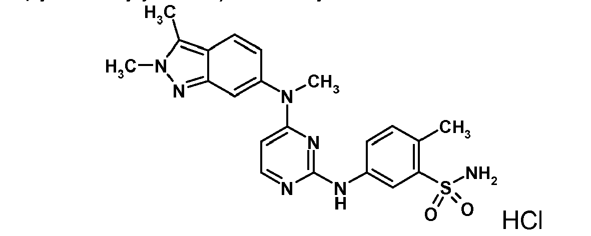

To a 1 L jacketed reactor was charged with acetonitrile (563 mL), water (188 mL) the monohydrochloride salt of Example 1, procedure 6 (50 g, 0.105 mol). The solution was stirred and heated to the jacket temperature at 85°C and a clear solution was obtained. The solution was then cooled down to 45°C and held for 90 minutes to cause crystallization of the hydrate After the 90 min hold, the solution was cooled down to 0°C, held for an hour and then filtered through a filter-dryer. The filtered solids were then washed with acetonitrile (200 mL X 1 ) at 0°C. The solids were blown in the filter-dryer with nitrogen at 25°C until the LOD was less than 25%. Acetonitrile (300 mL) was charged to the solids in filter-dryer, and stirred at 60°C for at least 8 hours or until the form conversion was complete (no monohydrate remaining) as observed by DATR to form 5-({4-[(2,3-dimethyl-2H-indazol-6-yl)methylamino]-2-pyrimidinyl}amino)-2-methylbenzenesulfonamide monohydrochloride anhydrate. The contents of the filter-dryer were cooled to ∼30°C, and the filtrate was pushed off using nitrogen pressure. The filtercake was blown with nitrogen at ∼60 °C under vacuum until the LOD was less than 0.5%. The contents were cooled to 20 °C yielding 37.5 g (75%) of 5-({4-[(2,3-dimethyl-2H-indazol-6-yl)methylamino]-2-pyrimidinyl}amino)-2-methylbenzenesulfonamide monohydrochloride anhydrate.

### Biological Data

### Example 2:

### Clinical Study of Orally Administered pazopanib in Patients with Solid Tumors

Sixty-three patients (pts) with cancer were treated with pazopanib three times a week, once (qd) or twice (bid) daily in a dose escalation scheme. The amount and frequency of administration of pazopanib to a representative number of those pts is summarized below in Table 1. Pts were evaluated monthly and treated until disease progression or intolerable side effects. Clinical response was determined every 8 wks. Safety and pharmacodynamics were separately assessed.

**Table 1**

| **Dose (mg)** | **Dosing Regimen** | **Number of Subjects Receiving pazopanib** |
|---|---|---|
| 100 | 3 x week (M, W, F) | 2 |
| 50 | QD | 9 |
| 50 | 3 x week (M, W, F) | 7 |
| 100 | QD | 3 |
| 200 | QD | 3 |
| 300 | BID | 4 |
| 400 | QD | 4 |
| 500 | QD | 1 |
| 600 | QD | 3 |
| 800 | QD | 8 |
| 1000 | QD | 2 |
| 1400 | QD | 3 |
| 2000 | QD | 2 |

Patients that achieved peak plasma concentrations of pazopanib of ≥40uM also demonstrated a clinical benefit, regardless of tumor type. Preliminary representative patient data are shown below in Table 2.

**Table 2**

| Tumor Type | Dose (mg) | Response | Duration on Duration on pazopanib (weeks) |
|---|---|---|---|
| Hurthle Cell | 50-800 qd | SD (MR) | 101 |
| Neuroendocrine | 1000 qd | PR^{#} | 38 |
| Neuroendocrine | 300 bid | MR | 50+ |
| Rectal carcinoid | 800 qd | SD | 40+ |
| GIST | 600-800 qd | SD | 60 |
| Adeno lung | 200-800 qd | SD | 55 |
| Chondrosarcoma | 2000-800 qd | SD (MR) | 90+ |
| Chondrosarcoma | 400-800 qd | SD | 31 |
| Leiomyosarcoma | 50-800 qd | SD | 34 |
| Melanoma | 50-400 qd | SD | 36 |
| Renal cell carcinoma | 1400-800 qd | SD (MR) | 40 |
| Renal cell carcinoma | 2000 qd | SD (MR) | 24 |
| Renal cell carcinoma | 800 qd | SD (MR)* | 19 |
| Renal cell carcinoma | 300 bid | PR | 52+ |
| Renal cell carcinoma | 300 bid | MR | 40+ |
| Renal cell carcinoma | 800 qd | SD | 12+ |

| | | | |
|---|---|---|---|
| ^{#} Preliminary data not yet confirmed ^{*} Patient withdrawn from study | | | |

Although specific embodiments of the present invention are herein illustrated and described in detail, the invention is not limited thereto. The above detailed descriptions are provided as exemplary of the present invention and should not be construed as constituting any limitation of the invention. Modifications will be obvious to those skilled in the art, and all modifications that do not depart from the spirit of the invention are intended to be included with the scope of the appended claims.

## Claims

1. A compound of formula (1): 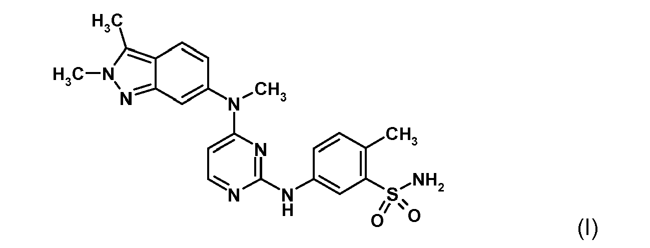 or pharmaceutically acceptable salt thereof for the treatment of a susceptible cancer, wherein said susceptible cancer is thyroid cancer.

2. A compound of claim 1 wherein said compound is the compound of formula (I')

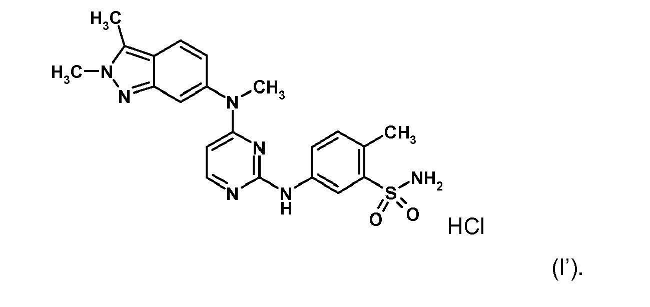

3. A compound of claim 1 wherein said compound is the compound of formula (1")

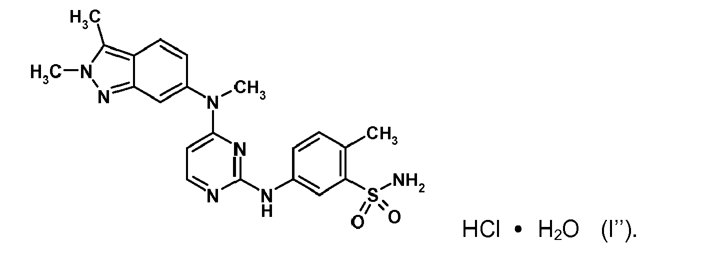

4. Use of a compound as defined in claims 1-3 in the manufacture of a medicament for the treatment of a susceptible cancer, wherein said susceptible cancer is thyroid cancer.

5. A pharmaceutical composition comprising a compound as defined in claims 1-3 together with a pharmaceutically acceptable carrier for the treatment of a susceptible cancer thereof, wherein said susceptible cancer is thyroid cancer.
